# EUROPÄISCHE PATENTSCHRIFT

(11) **EP 1 419 133 B1**
(45) Veröffentlichungstag und Bekanntmachung des Hinweises auf die Patenterteilung: **20.12.2006**
(21) Anmeldenummer: 02796225.7
(22) Anmeldetag: 13.08.2002
(51) Int. Cl.: C07C 68/06, C07C 68/08, C07C 69/96

(54) **VERFAHREN ZUR HERSTELLUNG VON DIALKYLCARBONATEN**
METHOD FOR THE PRODUCTION OF DIALKYL CARBONATES
PROCEDE DE FABRICATION DE DIALKYCARBONATES

(30) Priorität: 21.08.2001 DE 10140846
(43) Veröffentlichungstag der Anmeldung: 19.05.2004
(73) Patentinhaber: Cognis IP Management GmbH, 40589 Düsseldorf (DE)
(72) Erfinder: RIDINGER, Richard, 40789 Monheim (DE); YÜKSEL, Levent, 40597 Düsseldorf (DE); FIEG, Georg, 40822 Mettmann (DE)
(86) Internationale Anmeldenummer: PCT/EP2002/009042
(87) Internationale Veröffentlichungsnummer: WO 2003/018527

(56) Entgegenhaltungen:
- EP-A- 0 089 709
- EP-A- 0 393 749
- WO-A-97/47583

## Beschreibung

Die Erfindung befindet sich auf dem Gebiet der Kosmetik und betrifft ein Verfahren zur Herstellung von Ölkörpem von Typ der Dialkylcarbonate, bei dem Produkte mit verbesserten geruchlichen Eigenschaften erhalten werden.

### Stand der Technik

Dialkylcarbonate stellen interessante neue Ölkörper für die Kosmetik dar und werden üblicherweise durch Umesterung von Dimethyl- oder Diethylcarbonat mit längerkettigen Fettalkoholen hergestellt. Besonders bevorzugt sind Herstellung und Einsatz von Dioctylcarbonat, das unter der INCI-Bezeichnung Dicaprylyl Carbonate und der Marke Cetiol® CC (Cognis Deutschland GmbH) im Handel ist [vgl. **WO 97/47583** (Cognis)]. Von Nachteil ist jedoch, dass die Carbonate einen schweißigen Eigengeruch besitzen, so dass es bei ihrer Verwendung in der Kosmetik eine Parfümierung erforderlich ist. Eine für diese Fälle übliche Wasseniampfdesodorierung hat sich als erfolglos erwiesen.

Die Aufgabe der vorliegenden Erfindung hat folglich darin bestanden, Dialkylcarbonate zur Verfügung zu stellen, die gegenüber dem Stand der Technik eine verbesserte Geruchsqualität aufweisen, um auf diesem Wege deren Einsatz in der Kosmetik zu erleichtern.

### Beschreibung der Erfindung

Gegenstand der Erfindung ist ein Verfahren zur Herstellung von Dialkylcarbonaten der Formel (I),

**R¹OCOOR²** **(I)**

in der R¹ und R² unabhängig voneinander für lineare oder verzweigte Kohlenwasserstoffreste mit 6 bis 22 Kohlenstoffatomen und 0 oder 1 bis 3 Doppelbindungen steht, durch Umesterung von C₁-C₄-Dialkylcarbonaten mit C₆-C₂₂-Alkoholen, bei dem man
(a) das rohe Umesterungsgemisch einer ersten Rektifikation unterwirft und dabei die leichtsiedenden Verunreinigungen abdestilliert,
(b) das so erhaltene Sumpfprodukt einer zweiten Rektifikation unterwirft und dabei die schwersiedenden Verunreinigungen abtrennt, und
(c) das so erhaltenen Destillat schließlich in einer Desodorierungskolonne mit Hilfe von Wasserdampf oder Inertgasen von mittelsiedenden Verunreinigungen, insbesondere Geruchsträgern befreit.

Der Erfindung liegt die Erkenntnis zugrunde, das die geruchliche Beeinträchtigung der Dialkylcarbonate auf ganz verschiedene Verunreinigungen zurückgeht, die nur durch unterschiedliche Verfahrensmaßnahmen entfernt werden können. Überraschenderweise wurde gefunden, dass die Kombination aus zwei Rektifikations- und einem Desodierungsschritt endlich ein Produkt liefert, das geruchlich einwandfrei ist und ohne zusätzliche Parfümierung in der Kosmetik eingesetzt werden kann.

### Rektifikation

Im Zuge der zweistufigen Rektifikation werden im ersten Schritt die unerwünschten leichtsiedenden leichtsiedenden Verunreinigungen abgetrennt. Das Sumpfprodukt wird dann einer zweiten Rektifikation unterworfen, bei der man die schwersiedenden Verunreinigungen im Sumpf belässt und nur das Destilat weiterverwendet. Als besonders vorteilhaft, weil die Einsatzstoffe wenig schädigend, hat es sich erweisen, das rohe Umesterungsgemisch und/oder das zwischenzeitlich erhaltene Destillat mit Hilfe einer Kombination aus Dünnschicht- und Fallfilmverdampfer zu verdampfen. Die Rektifikation selbst wird insbesondere in Kolonnen mit strukturierten Packungen durchführt, welche vorzugsweise einen Druckverlust von weniger als 1-2 mbar/m aufweisen. Üblicherweise wird die erste Rektifikation bei Temperaturen (Sumpf) im Bereich von 180 bis 250°C und einem verminderten Druck (Kopf) von 0,01 bis 10 mbar, vorzugsweise bei Temperaturen (Sumpf) von 180 bis 200°C und einem verminderten Druck (Kopf) von 1 bis 10 mbar durchgeführt. Die zweite Rektifikation erfolgt üblicherweise bei Temperaturen (Sumpf) von 150 bis 250°C und einem verminderten Druck (Kopf) von 0,01 bis 0,5 mbar, vorzugsweise bei Temperaturen (Sumpf) von 150 bis 180°C und einem verminderten Druck (Kopf) von 0,01 bis 0,5 mbar.

### Desodorierung

Die Desodorierung wird in der Regel in einer Kolonne bei einem verminderten Druck von 10 bis 100 mbar mit Hilfe von Wasserdampf oder Inertgasen, vorzugsweise Stickstoff durchgeführt.

Die Aufarbeitung eines technischen Dioctylcarbonates wurde in zwei stofflich gekoppelten Rektifikationskolonnen mit jeweils 0,5 m EX-Packung der Sulzer AG sowie einer Desodorierungskolonne durchgeführt. Die Kolonnendurchmesser betrugen 30 mm. Das rohe Umesterungsgemisch wurde über eine Kombination von Dünnschicht- und Fallfilmverdampfer verdampft und auf die erste Rektifikationskolonne aufgegeben, wobei der Kopfdruck 5 mbar, die Sumpftemperatur 185 °C, der Druckverlust ca. 9 mbar und das Rücklaufverhältnis 5 betrug. Es wurden 43,5 Gew.-% leichtsiedendes Destillat und 56,5 Gew.-% Wertprodukt im Sumpf erhalten. Anschließend wurde das Sumpfprodukt auf die gleiche Weise verdampft und auf die zweite Rektfikationskolonne aufgegeben, wobei der Kopfdruck 0,1 mbar, die Sumpftemperatur 176 °C, der Druckverlust ca. 9 mbar und das Rücklaufverhältnis 3 betrug. Die anschließende Desodorierung erfolgte bei 100 °C in einer separaten Kolonne mit Stickstoff. Es wurde ein Produkt erhalten, welches sich auch nach 6-monatiger Lagerung als völlig geruchsfrei erwies.

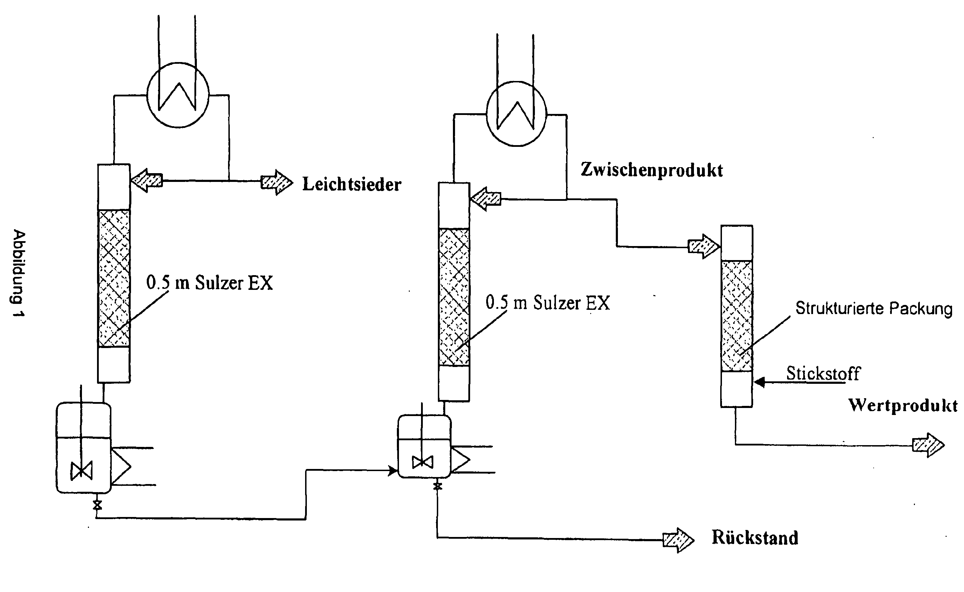

Die Abbildung 1 zeigt ein Fließschema des Verfahrens.

## Patentansprüche

1. Verfahren zur Herstellung von Dialkylcarbonaten der Formel (I),
**R¹OCOOR²** **(I)**
in der R¹ und R² unabhängig voneinander für lineare oder verzweigte Kohlenwasserstoffreste mit 6 bis 22 Kohlenstoffatomen und 0 oder 1 bis 3 Doppelbindungen steht, durch Umesterung von C1-C4-Dialkylcarbonaten mit C₆-C₂₂-Alkoholen, bei dem man
(a) das rohe Umesterungsgemisch einer ersten Rektifikation unterwirft und dabei die leichtsiedenden Verunreinigungen abdestilliert,
(b) das so erhaltene Sumpfprodukt einer zweiten Rektifikation unterwirft und dabei die schwersiedenden Verunreinigungen abtrennt, und
(c) das so erhaltene Destillat schließlich in einer Desodorierungskolonne mit Hilfe von Wasserdampf oder Inertgasen von mittelsiedenden Verunreinigungen befreit.

2. Verfahren nach Anspruch 1, **dadurch gekennzeichnet, dass** man das rohe Umesterungsgemisch und/oder das zwischenzeitlich erhaltene Destillat mit Hilfe einer Kombination aus Dünnschicht- und Fallfilmverdampfer verdampft.

3. Verfahren nach den Ansprüchen 1 und/oder 2, **dadurch gekennzeichnet, dass** man die Rektifikation in Kolonnen mit strukturierten Packungen durchführt.

4. Verfahren nach Anspruch 3, **dadurch gekennzeichnet, dass** man Kolonnen mit strukturierten Packungen einsetzt, die einen Druckverlust von weniger als 1-2 mbar/m aufweisen.

5. Verfahren nach mindestens einem der Ansprüche 1 bis 4, **dadurch gekennzeichnet, dass** man die erste Rektifikation bei Temperaturen (Sumpf) im Bereich von 180 bis 250 °C und einem verminderten Druck (Kopf) von 0,01 bis 10 mbar durchführt.

6. Verfahren nach mindestens einem der Ansprüche 1 bis 5, **dadurch gekennzeichnet, dass** man die zweite Rektifikation bei Temperaturen (Sumpf) von 150 bis 250 °C und einem verminderten Druck (Kopf) von 0,01 bis 0,5 mbar durchführt.

7. Verfahren nach mindestens einem der Ansprüche 1 bis 6, **dadurch gekennzeichnet, dass** man die Desodorierung bei einem verminderten Druck von 10 bis 100 mbar durchführt.

## Claims

1. A process for the production of dialkyl carbonates corresponding to formula (I):
**R¹OCOOR²** **(I)**
in which R¹ and R² independently of one another represent linear or branched hydrocarbon radicals containing 6 to 22 carbon atoms and 0 or 1 to 3 double bonds,
by transesterification of C₁₋₄ dialkyl carbonates with C₆₋₂₂ alcohols, **characterized in that**
(a) the crude transesterification mixture is subjected to a first rectification in which the low-boiling impurities are distilled off,
(b) the "bottom" product thus obtained is subjected to a second rectification in which the high-boiling impurities are removed and
(c) finally, in a deodorizing column, the distillate thus obtained is freed from medium-boiling impurities, either with steam or with inert gases.

2. A process as claimed in claim 1, **characterized in that** the crude transesterification mixture and/or the distillate obtained as intermediate product is evaporated in a combination of thin-layer and falling-film evaporators.

3. A process as claimed in claims 1 and/or 2, **characterized in that** the rectification is carried out in columns with structured packings.

4. A process as claimed in claim 3, **characterized in that** columns with structured packings which have a pressure loss of less than 1-2 mbar/m are used.

5. A process as claimed in at least one of claims 1 to 4, **characterized in that** the first rectification is carried out at temperatures (bottom) of 180 to 250°C and under a reduced pressure (head) of 0.01 to 10 mbar.

6. A process as claimed in at least one of claims 1 to 5, **characterized in that** the second rectification is carried out at temperatures (bottom) of 150 to 250°C and under a reduced pressure (head) of 0.01 to 0.5 mbar.

7. A process as claimed in at least one of claims 1 to 6, **characterized in that** the deodorization is carried out under a reduced pressure of 10 to 100 mbar.

## Revendications

1. Procédé de préparation de carbonates de dialkyle répondant à la formule (I),
R¹ OCOOR² (I)
dans laquelle R¹ et R² représentent, indépendamment l'un de l'autre, des radicaux hydrocarbonés linéaires ou ramifiés ayant 6 à 22 atomes de carbone et 0 ou 1 à 3 double(s) liaison(s), par transestérification de carbonates de dialkyle en C₁ - C₄ avec des alcools en C₆- C₂₂, selon lequel
(a) on soumet le mélange de transestérification brut à une première rectification et on élimine ainsi les impuretés à bas point d'ébullition,
(b) on soumet le produit de bas de colonne ainsi obtenu à une deuxième rectification et on sépare ainsi les impuretés à point d'ébullition élevé, et
(c) on débarrasse finalement le distillat ainsi obtenu des impuretés à point d'ébullition moyen, dans une colonne de désodorisation à l'aide de vapeur d'eau ou de gaz inertes.

2. Procédé selon la revendication 1,
**caractérisé en ce qu'**
on évapore le mélange de transestérification brut et/ou le distillat intermédiaire à l'aide d'une combinaison d'évaporateur à couche mince et d'évaporateur à film tombant.

3. Procédé selon les revendications 1 et/ou 2,
**caractérisé en ce qu'**
on effectue la rectification dans des colonnes à garnissages structurés.

4. Procédé selon la revendication 3,
**caractérisé en ce qu'**
on utilise des colonnes à garnissages structurés qui présentent une perte de pression inférieure à 1 - 2 mbar/ m.

5. Procédé selon au moins l'une des revendications 1 à 4,
**caractérisé en ce qu'**
on effectue la première rectification à des températures (bas de colonne) situées dans la plage de 180 à 250 °C et sous une pression réduite (tête de colonne) de 0,01 à 10mbar(s).

6. Procédé selon au moins l'une des revendications 1 à 5,
**caractérisé en ce qu'**
on effectue la deuxième rectification à des températures (bas de colonne) de 150 à 250 °C et sous une pression réduite (tête de colonne) de 0,01 à 0,5 mbar.

7. Procédé selon au moins l'une des revendications 1 à 6,
**caractérisé en ce qu'**
on effectue la désodorisation sous une pression réduite de 10 à 100 mbars.
